# EUROPEAN PATENT APPLICATION

(11) **EP 3 763 334 A1**
(43) Date of publication of application: **13.01.2021**
(21) Application number: 18908776.0
(22) Date of filing: 09.03.2018
(51) Int. Cl.: A61F 2/32, A61F 2/34, A61F 2/36, A61L 27/14, A61L 27/04, C08L 23/06

(54) **HIP REPLACEMENT PROSTHESIS WITH HIGHLY CROSS-LINKED POLYETHYLENE HEAD**

(71) Applicant: Pérez Núñez, Rafael Eduardo, Bogotá, 110221 (CO)
(72) Inventor: Pérez Núñez, Rafael Eduardo, Bogotá, 110221 (CO)
(74) Representative: Linage González, Rafael
(86) International application number: PCT/IB2018/051565
(87) International publication number: WO 2019/171158

(57) **Abstract**

This invention corresponds to a prosthesis for total hip or hip resurfacing replacement, comprising a prosthetic femoral head made of highly cross-linked polyethylene, with a diameter ranging from 38 mm to 64 mm, to articulate with a cup or acetabular component made of metal. When the invention applies for total hip replacement, the polyethylene head includes a metal or ceramic core, which contains inside the female counterpart (14) to mate with the male counterpart (13) of a Morse taper located at the upper end of the femoral component. Said core has a safe intra-prosthetic anti-rotational locking mechanism to assemble the polyethylene head. The use of this type of head for total hip replacement, articulated with an ultra-polished acetabular cup, reduce the risk of dislocation, transmits less angular and torque forces to the Morse taper than large metal heads, and avoids problems related to the metal-metal bearing or with the use of large metal heads with thin polyethylene.

When the invention relates to hip resurfacing replacement, the highly cross-linked polyethylene femoral head has a metal stem integrated into a metal-back (152), safely assembled to the polyethylene head. Using these types of heads for hip resurfacing replacements eliminates the problems associated with metal-on-metal resurfacing replacement.

## Description

### FIELD OF THE INVENTION

The present invention relates to total hip or resurfacing hip replacement prosthesis characterized by a femoral head prosthesis made of highly cross-linked polyethylene, with diameter ranging from 38 mm to 64 mm, to articulate with a cup or acetabular component made of metal.

When the invention applies to total hip replacement the polyethylene head engages the conical portion situated in the upper end of the femoral stem, which corresponds to the male component of a Morse taper, through a metal connector core, which is stably assembled in the head by means of a locking mechanism and which contains the female counterpart of the Morse taper. The use of a large, highly cross-linked polyethylene head associated with a connector core allows for a greater arc of movement, reducing the risk of dislocation while transmitting less angular and torque forces to the Morse taper than large metal heads. All this, without the biological problems related to the metal-metal bearing combination or the use of a thin polyethylene.

When the invention is applied to resurfacing hip replacement, the prosthetic femoral head made of highly cross-linked polyethylene extension an extension or distal stem that allows fixation directly to the femoral neck with bone cement, preserving the advantages of bone preservation, load transmission, stability and ease of revision of resurfacing prostheses.

### BACKGROUND OF THE INVENTION

Total hip replacement is a surgery with a very high success rate in relieving pain and improving the functional capacity of patients with osteoarthritic processes in the hip joint. Current implant cementation techniques, the use of porous-coated implants for bone ingrowth, and the introduction of new low-wear materials prevent early loosening of the implant and provide long-lasting fixation. However, prosthetic instability continues to be a frequent cause of reoperation.

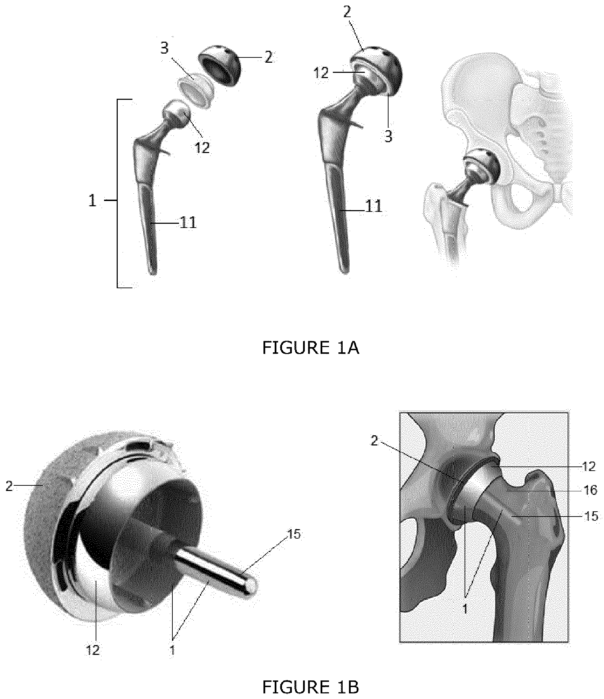

A total hip replacement prosthesis is composed of an acetabular component (2) and a femoral component (1), as shown in Figure 1A. Both can be fixed to the patient's bone with bone cement (cemented prostheses) or without cement (uncemented prostheses). In the latter, fixation is achieved through the use of porous-coated implants that allow bone growth inside it or on the irregularities of its surface. This process is called osteo-integration.

In the case of cemented implants, there is an immediate stability after the cement has set, while in uncemented implants it is necessary to achieve enough initial stability, by press fitting of the implants or with supplementary screws fixation in the acetabulum (2), which allows the osteo-integration of the implants, which is ultimately the phenomenon that provides long-term stability.

Currently, the most commonly used acetabular components are uncemented and modular, where the porous coating for osteo-integration varies in its properties and composition depending on the manufacturer. These components are made up of an external metal shell or acetabular cup, which is fixed in the native acetabular cavity by press-fit, with the option of additional fixation with screws. An internal modular polyethylene or polyethylene liner (3) is attached to the acetabular cup (2), which presents on its articular side a hemispherical cavity to receive the metallic or ceramic femoral head (12) as shown in the Figure 1A. These heads have a diameter of 22, 26, 28, 32, 36 or more millimeters.

The cemented femoral component of the total hip replacement is a smooth-surface or minimally rough metal stem that is fixed in the medullary canal of the femur by bone cement (poly-methyl methacrylate), while the uncemented femoral component (1) is covered with a surface for osteo-integration, which varies in its extension, properties and composition, according to the manufacturer.

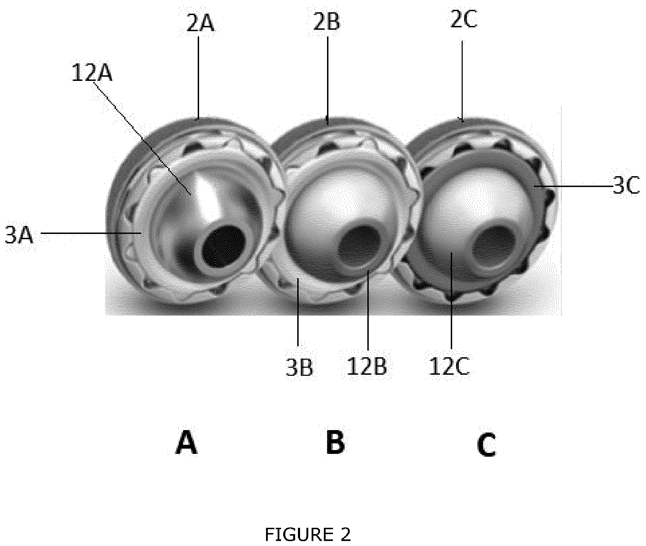

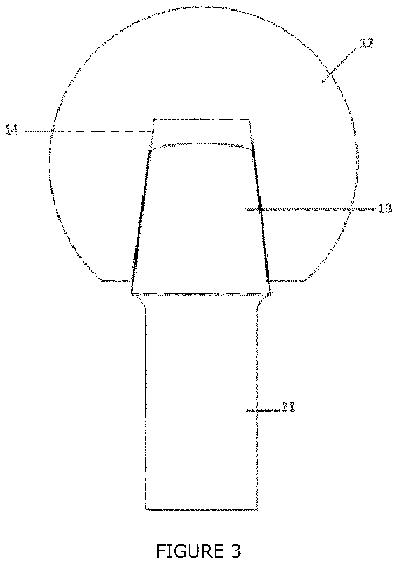

The femoral component (1) articulates with the acetabular polyethylene (3) through a modular head (12), which can be metallic (12A) or ceramic (12B and 12C), as shown in Figure 2. These heads (12) are assembled to the stem (11) by means of a Morse taper system consisting of a male component (13), located at the upper conical end of the femoral stem (11), and a female component (14), corresponding to a conical cavity of the femoral head (12), with minimum tolerance between the angle of the cone (13) of the stem and the angle of the conical cavity (14) in the head (12), to obtain a wide contact between the surfaces in order to achieve a firm and durable fixation, as illustrated in Figure 3.

On the other hand, resurfacing hip replacement is a hip arthroplasty in which a metallic acetabular component with a large inner diameter and ultra-polished internal surface (2) is placed with a femoral component (1), which replaces only the articular surface of the femoral head (12). Currently, a metallic femoral component (1) is used with a stem (15) for fixation in femoral neck (16), as shown in Figure 1B. The advantages of this type of implant are that the bone resection is less, since the femoral neck does not have to be removed, the anatomy of the hip is better restored, the transmission of loads to the proximal femur is optimized, there is less risk of dislocation and revision surgery becomes easier.

Either, total hip replacement and resurfacing hip replacements prostheses had undergone an evolutive process aimed at improving their performance and avoiding wear of their components in order to offer the patient a more efficient and durable implant. In the search for improvement, it has been established that a relevant variable is the material from which the surfaces of the prosthesis that support the load and the movement of the joint are made of.

The history of joint replacements begins with Smith-Peterson in the 20 of the last century, by replacing the head of the femur with an implant, preserving the native acetabulum. Initially molded glass heads were used but because of their fragility they were replaced by metal alloys implants, of which the most successful was the molded metallic cup of Smith- Peterson, manufactured in Vitallium, a chromium-cobalt-molybdenum alloy. However, pain relief with these implants was not complete, since limbs could not be equalized and mobility was insufficient.

In the decade of the 30' of the 20 century, Philip Wiles at Middlesex Hospital in London implanted the first total hip replacement, replacing both sides of the joint with a metal-metal prosthesis. Subsequently, G.K. McKee in Norwich, England used different uncemented metal on metal implant designs which failed by early loosening.

From the introduction of the cement in 1953 the results improved and in the subsequent decade the metal-metal of McKee-Farrar prosthesis was the most used total hip replacement. On the otherhand, John Charnley at Wrightington, Britain, believed that the metal-metal joint worked with high frictional forces, which would lead to early component loosening in the short term anyway. As an alternative to the metal-metal prosthesis, Charnley used in the early 50' of the last century Teflon-Teflon resurfacing replacements (femoral head and acetabular resurfacing with Teflon), but this type of implant was abandoned due to the rapid wear of both components.

Later, in the late 50' and the early 60' of the last century a total hip replacement with metal femoral head and a teflon acetabular component were used, but these implants also failed due to wear of the Teflon and bone resorption around the implant. Since 1962, Charnley replaced the teflon acetabular cup for a cup made of High density polyethylene (HDP), and used 22. 25 mm heads to decrease the volumetric wear associated with larger heads and the results improved considerably. Some years later, the high-density polyethylene was changed to Ultra-high molecular weight polyethylene (UHMWPE). This combination (metal-UHMWPE) was for many years the most used Total hip prosthesis in the world.

Simultaneously with the advances in total hip replacement implants, in the 70' of the 20 century an interest for surface replacements arose again, arguing greater preservation of bone in the femur, better transmission of loads, greater stability and the ease of revision to a total hip prosthesis.

Later, resurfacing hip replacements emerged with cemented metal head and polyethylene cups, being the implants from Wagner and Amstutz the most used but after seven years of follow - up, it was established that the failure rate of the Wagner prosthesis was greater than 50%, because of the high volume of polyethylene wear that occurred at the joint with a large metal head, so these type of prostheses were not used anymore.

In the 90' of the last century, it is evident that the phenomena of peri-prosthetic bone resorption (osteolysis), which develops around cemented and cementless implants at medium and long term follow-up when a metal head is used against a polyethylene cup are mainly related to wear debris of the acetabular cup, so a search for new materials began and the use of metal on metal articulation resurged with lower surface roughness and proper clearance between the femoral head and the acetabulum in order to improve lubrication and be able to eliminate the polyethylene. These same principles were applied to a new resurgence of metal-to-metal joint resurfacing replacements and the use of larger diameter femoral heads in metal-to-metal total prostheses.

In this decade greater attention was also paid to the use of ceramic heads and ceramic acetabular inserts, and highly cross-linked polyethylene (HCLPE) acetabular inserts became available which are hardened polyethylene through irradiation process and heat treatment, offering improved wear resistance compared with conventional polyethylene (Ultra high molecular weight polyethylene).

Currently, the most widely used pairs of prosthetic movement surfaces are those shown in Figure 2, which include metal heads against highly cross-linked polyethylene (2A), ceramic head against highly cross-linked polyethylene (2B) and ceramic head against a ceramic acetabular insert. (2C). The latter is the one that shows less wear both in-vitro and in-vivo.

With the use of more wear-resistant materials, such as metal-metal, ceramic-ceramic, ceramic-HCLPE, metal-HCLPE pairs of friction, the use of larger diameter heads is introduced almost simultaneously in total hip replacement with the aim of improving the safe range of motion of the joint and decrease the incidence of prosthetic dislocation (disassembly of the prosthetic femoral head from its acetabular counterpart), which has been one of the main complications of total hip replacement.

The incidence of prosthetic dislocation in primary total hip replacements is between 0,5 % and 6,9%, and increases to 15% in cases of revision surgeries (implant exchange surgery. Its cause is multifactorial, and variables such as surgical approach, primary diagnosis and head size influence the occurrence of this complication. The incidence of prosthetic dislocation using a posterior approach, decreased from 12% with 22 mm heads to 3,8% with 32 mm heads *(*Berry et al. Effect of the femoral head diameter and operative approach on risk of dislocation after primary Total hip arthroplasty, Journal of Bone and Joint Surgery, 2005; 87-A (11): 2456*), (*Phillips et al. Incidence rates of dislocation, pulmonary embolism and deep infection during the first six months after elective total hip replacement. Journal of Bone and Joint Surgery, 2003; 85- A (1): 20*).* Other important factors that lead to prosthetic dislocation are associated with the patient, as cultural, religious and recreational activities that require bringing the joint into extreme ranges of motion increase the risk of dislocation, even in properly selected and implanted prostheses.

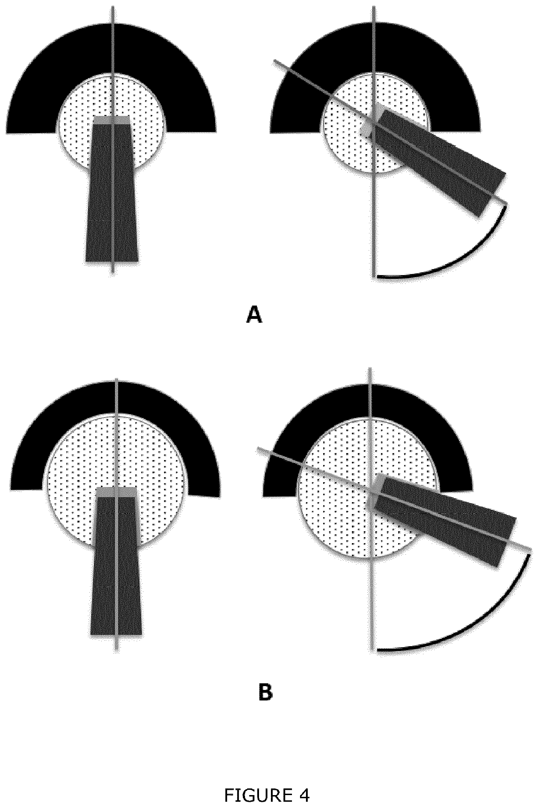

In principle, it is considered that a larger prosthetic head increases the stability of the total hip replacement, because it increases the head-neck diameter ratio of the prosthesis and increases the impingement free range of motion, as evidenced in the Figures 4 A and B. In addition, a larger diameter of the head increases the distance the center of rotation of the prosthetic head has to displace in order to disengage from the cavity. Likewise, as this displacement is greater, the elongation of the soft tissues for dislocation to occur has to be greater, since they act as restrictors for displacement and dislocation.

However, the use of large heads for total hip replacement has had several drawbacks. The first is that to use a larger diameter head, without varying the diameter of the acetabular component, a thinner polyethylene or ceramic inserts must be used, and thin components have been shown to be at increased risk of failure due to accelerated wear or material fatigue.

Likewise, it has been found that the heads with a larger diameter, despite showing less linear wear, produce greater volumetric wear of the acetabular polyethylene. Thus, it is considered that the volume of particles generated in the joint and its negative consequences may be greater. This wear is less when low wear acetabular inserts (3) such as HCLPE or ceramics (Figure 2) are used. However, these components are much more brittle than conventional polyethylene and have a higher risk of material failure, especially if their thickness is thin *(*Tower et al, Rim Cracking of the cross-linked Longevity acetabular liner after total hip arthroplasty. Journal of Bone and Joint Surgery, 2007; 89-A (10): 2212*).*

On the other hand, a head with a larger diameter could increase the torque and bending forces transmitted to the Morse taper, which joins the head with the femoral component, increasing the risk of local corrosive processes and the release of ions and metallic particles, with their local and systemic consequences. *(*Cooper et al. Corrosion at the Head-Neck Taper as a Cause for Adverse Local Tissue Reactions After Total Hip Arthroplasty. Journal of Bone and Joint Surgery, 2012; 94-A (18): 1655*.*

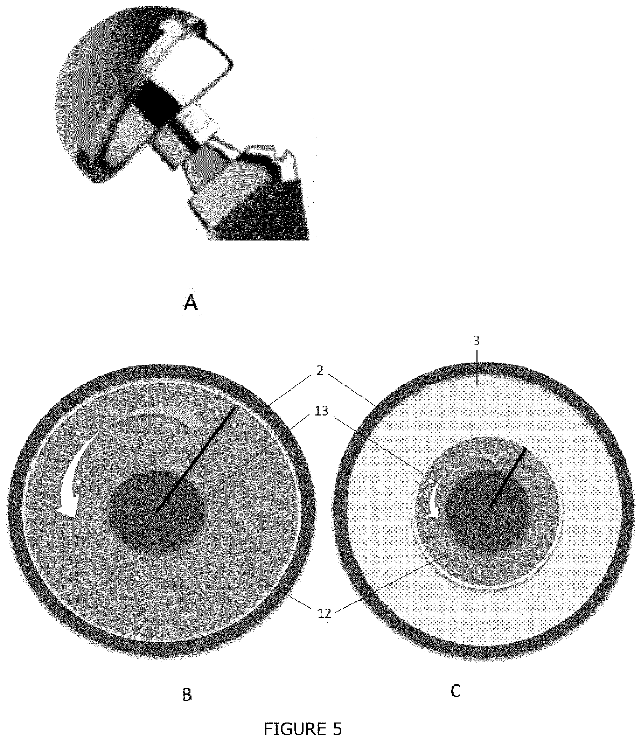

With metal on metal total hip replacement implants, in which the larger diameter metal femoral head articulates directly with an ultra-polished metal acetabular component (Figure 5A), there were no fragility issues and the results in relation to short-term loosening and dislocation were good, but in the medium term, drawbacks appeared due to high levels of metal ions in the blood and catastrophic adverse local reactions, with large destruction of peri-prosthetic tissues, especially when big heads were used. *(*Meyer et al. Corrosion at the Cone/Taper Interface Leads to failure of Large-diameter metal-on-Metal Total Hip Arthroplasties. Clinical Orthopedics and Related Research, 2012; 470 (11): 3101*);* Langton et al. Taper Junction Failure in Large Diameter Metal on Metal Bearings. Bone Joint Research 2012, Vol. 1 (4): 56*).* In addition to the aforementioned failures, metal ion drawbacks and adverse reactions were also reported in metal-metal implants for resurfacing replacements, causing the use of both types of replacement devices to be abandoned or to be used only with very limited indications. *(*Langton et al. Accelerating failure rate of ASR total hip replacement. J. Bone Joint Surg (Br), 2011; 93B (8): 1011*;* Hart et al. Pseudotumors in Association with Well-Functioning Metal on Metal Hip Prostheses. J Bone Joint Surg (Am), 2012; 94: 317*).*

The cause of these problems seems to be a combination of punctual areas of concentration of loads on the articular surface of the implants with poorly positioned acetabular components (edge loading) and the generation of larger amounts of corrosion products at the Morse taper interface due to increased transmission of frictional torque and bending forces to the taper of the femoral component (Figure 5B and 5C) compared to small heads (Figure 5C), and even the development of instability in the interface between the head (12) and the femoral stem (11) (Figure 3). For these reasons, large-head for metal-on-metal surfaces, such as those shown in Figure 5A, have been abandoned.

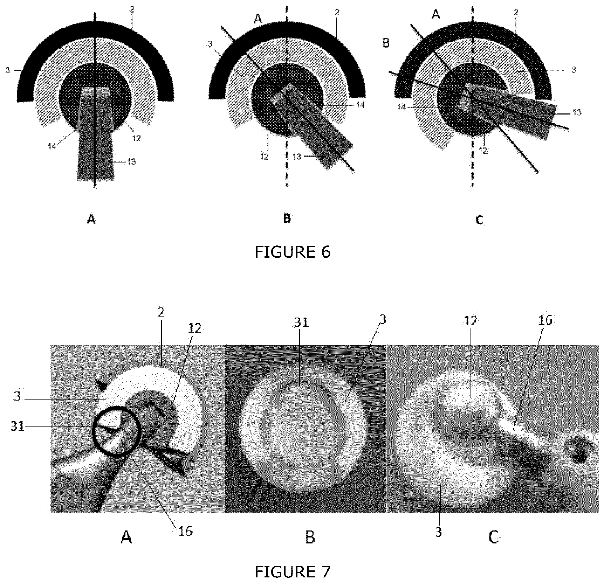

In an attempt to improve mobility and stability, without using the metal-on-metal combination, dual mobility cups were introduced, a novel and attractive concept shown in Figure 6A, 6B and 6C. This alternative consists of assembling a smaller diameter metal or ceramic head in a larger diameter polyethylene mobile acetabular insert, which in turn, articulates with a ultra-polished metal cup, which has an exterior surface for either cementing or bone ingrowth. In this way, two mobility sites are generated, that is, two joints, as shown in Figure 6. The main joint is given by the movement of the metal head (12) inside the polyethylene insert (3), and the secondary joint is represented by the movement between the mobile polyethylene (3) and the metal cup (2), thereby expanding the arc of movement.

It is important to clarify that the assembly of the metal head (12) in the polyethylene insert (3) is under pressure, using a capture mechanism to avoid dislocation. That is, the opening diameter of the polyethylene insert (3) is less than the diameter of the metal head (12), which requires some deformation of the polyethylene (3) during the assembly of the head, so for these type of implants the use of highly crosslinked polyethylene in not considered to be safe, even though they wear less, because they are more brittle to deforming forces.

With this type of implants, the incidence of prosthetic dislocation in general has been reduced. However, there is a 0 and 5% of patients who present what has been called intra-prosthetic dislocation, which refers to the decoupling of the metal head (12) from the polyethylene insert (3). This occurs due to wear of the polyethylene rim (31), which gives stability to the head (12), due to friction and repetitive impact with the femoral neck (16). The results of this wear are evidenced in Figures 7A, 7B and 7C. The given reasons have limited the use of this type of implants to elderly patients or those at high risk of dislocation *(*Hamadouche et al. Is a Cementless Dual Mobility Socket in Primary THA a reasonable option. Clinical Orthopedics and related research 2012 (470): 3048*.* Philippot et al. Survival of Cementless dual mobility sockets with a mean 17 years follow-up Rev. Chir Orthop Reaparatrice Appar Mot 2008, 94 (8): e23*).*

Although joint wear with dual mobility cups has been difficult to assess, wear of the mobile ultra-high molecular weight polyethylene (UHMWPE) insert (3) in these implants is apparently similar to that of conventional UHMWPE cups. *(*Gaudin et al. Equivalent wear performance of dual mobility bearing compared with standard bearing in total hip arthroplasty: An in vitro study, Int Orthop. 2017 Mar; 41 (3): 521-527*).* Furthermore, Loving et al. demonstrated in hip simulators that the highly cross-linked polyethylene in the dual mobility cups is more resistant to wear than the fixed polyethylene of conventional cups in situations of overload due to increased inclination of the metal cup (edge loading). *(*Loving and Cols. Dual Mobility Bearings Withstand Loading from Steeper Cup-inclinations Without Substantial Wear. Journal of Orthopedic Research, 2015 (March): 398*).*

In summary, we can conclude that, despite the many alternatives that have been tried and the improvements that have been made to implants, there is a need in the current state of the art of hip prostheses to have a prosthesis that allows the use of large heads in order to offer a greater range of movement and less risk of dislocation, without the problems derived from metal-metal combination and the use of very thin acetabular polyethylene.

During the decade of the 70s of the last century, the use of polyethylene heads articulated with metal cups in hip replacement was limited to surface replacement and was abandoned because it presented a high rate of loosening and wear of high-density polyethylene (HDPE).

Recently, Abhijit and Cols. conducted a finite element study comparing the stresses and displacements of the material between a stainless-steel metal head and an ultra-high molecular weight polyethylene head. This study concluded that the stresses on the heads are similar, but the displacement is greater with the polyethylene femoral heads, therefore they recommend using metal heads.

The present invention introducing a large HCLPE femoral head, not only seeks to overcome the problems raised above, but in the modality for total hip replacement it intend to reduce the transmission of torque forces to the Morse taper occurring with large metal heads and at the same time eliminates the site of movement between the metallic head and the polyethylene core prostheses in the double mobility type of implant, which is a source of polyethylene debris, both in the inner and at the periphery, which favors the occurrence of intra-prosthetic dislocation and osteolysis.

Likewise, the introduction of a polyethylene HCLPE head for hip surface replacement eliminates the risks of using metal-to-metal joints.

### DESCRIPTION OF THE FIGURES

Figure 1A. Prosthesis for total hip replacements with uncemented components.
Figure 1B. Prosthesis for resurfacing hip replacement.
Figure 2A. Cementless acetabular component with polyethylene insert and metal head.
Figure 2B. Cementless acetabular component with polyethylene insert and ceramic head.
Figure 2C. Cementless acetabular component with ceramic insert and ceramic head.
Figure 3. Scheme of a Morse taper.
Figure 4A. Arc of movement up to the impingement of the prosthetic femoral neck with the rim of the acetabular cup with a small head.
Figure 4B. Arc of movement up to the impingement of the prosthetic femoral neck with the rim of the acetabular cup with a large head.
Figure 5A. Metal-metal total hip prosthesis with large head.
Figure 5B. Scheme of transmission of the frictional torque to the Morse cone with large head.
Figure 5C. Scheme of transmission of the frictional torque to the Morse cone with small head.
Figure 6A. Diagram of double mobility cup with the femoral component (1) without flexion.
Figure 6B. Diagram of double mobility cup showing the initial movement (A) between the metal head (12) and the polyethylene insert (3).
Figure 6C. Diagram of the double mobility cup, showing the late movement (B) between the polyethylene core (3) and the metal cup (2), after the neck of the femoral component (1) hits the polyethylene rim (3).
Figure 7A. Shock of the femoral neck (16) with the rim (31) of the polyethylene insert (3).
Figure 7B. Wear of the capture rim (31) of the mobile polyethylene insert (3) by repetitive shock and friction.
Figure 7C. Intra-prosthetic dislocation: the metallic head comes out of the mobile insert of polyethylene due to wear of the rim.
Figure 8. Longitudinal section of the implant of the present invention for total hip replacement, where the head (12 A) is made of highly cross-linked polyethylene and has a metal core (17) coupled inside and fixed by means of a lid locking mechanism (171).
Figure 9. Cross section of the implant of the present invention, through AA line of Figure 8, with hexagonal metal core.
Figure 10A. Cross section of the implant of the present invention, where the head (12A) has a square shaped metal core (17).
Figure 10B. Cross section of the implant of the present invention, where the head (12A) has a metal core (17) of pentagonal shape.
Figure 10C. Cross section of the implant of the present invention, where the head (12A) has a metallic core (17) of heptagonal shape.
Figure 10D. Cross section of the implant of the present invention, where the head (12A) has a metal core (17) of octagonal shape.
Figure 11A. Longitudinal section of the prosthesis of the invention with a truncated cone shaped metal core (17) with anti-rotatory mechanism (172), and flanges as an axial locking mechanism (1711).
Figure 11B. Cross section of the implant of the present invention through the AA line of Figure 11 A, showing the anti-rotating mechanism.
Figure 11C. Longitudinal section of the implant with a truncated-cone shaped metal core (17) with an anti-rotational mechanism (172) and with a metal ring, as a securing mechanism (1712).
Figure 12A. Longitudinal section of the metallic core (17) to be assembled to the polyethylene head (12 A) without extension.
Figure 12B. Bottom view of the metallic core (17) to be assembled to the polyethylene head (12 A) without extension.
Figure 12C. Longitudinal section of the metal core (17) to be assembled to the polyethylene head (12 A) with extension (174).
Figure 12D. Bottom view of the metallic core (17) to be assembled to the polyethylene head (12 A) with extension (174).
Figure 13A. Clearance between the cup (2) and the head (12 A) where larger contact is polar (22).
Figure 13B Clearance between the cup (2) and the head (12 A) where their greatest contact is in the equatorial region (23).
Figure 14A. Longitudinal section of the implant of the present invention for hip resurfacing replacement, where the highly cross-linked polyethylene head (12B) is observed with a highly cross-linked polyethylene distal stem (15) attached to the femoral neck (16).
Figure 14B. Longitudinal section of the implant of the present invention for hip surface replacement, where the highly cross-linked polyethylene head (12B) with a distal polyethylene stem (15) is observed with an internal metal reinforcement (151), attached to the femoral neck (16).
Figure 15. Perspective view of the femoral component of the implant for hip surface replacement, showing a metal-backed (152) highly cross-linked polyethylene head (12B) with a metal stem (15).
Figure 16. Breakup view of the femoral component of Figure 15, where the highly cross-linked polyethylene head (12B) with a circumferential raised rim (12 3) and anti-rotational tabs (124) is observed; a metal backing (152) with a lower circumferential edge (153) having two notches (154), to receive the anti-rotation tabs (124); and a metal stem (15), which for the purposes of the figure has been separated from the metal backing (152).
Figure 17A. Longitudinal section of the femoral component of Figure 15 coupled to an acetabular component (2). The highly cross-linked polyethylene head (12B) is observed with the circumferential raised flange (123) fitted under the circumferential lower edge (153) of the metal backing (152); and the metal stem (15) engaged in the femoral neck (16).
Figure 17 B. Cross section of the implant of the present invention, through the AA line of Figure 17 A, showing the anti-rotation tabs (124) for securing the polyethylene fitted inside the notches (154) of the lower circumferential edge of the metal back (152).

### DETAILED DESCRIPTION OF THE INVENTION

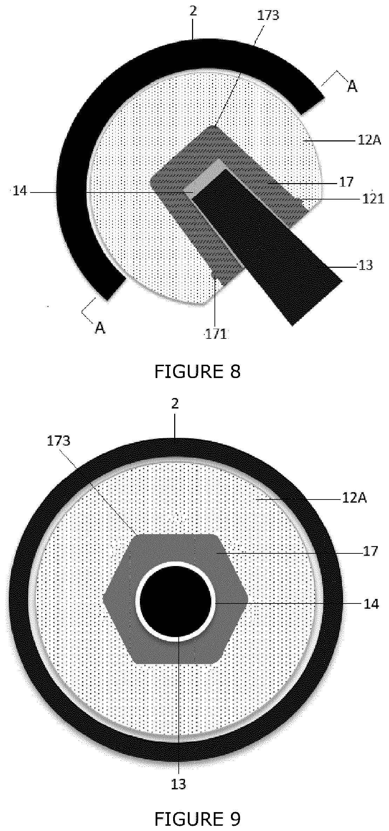

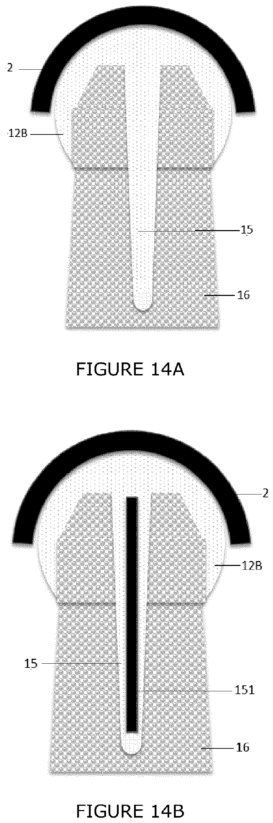

The present invention relates to a prosthesis which is characterized by comprising one head of highly cross-linked polyethylene (12A, 12B) of 38 mm to 64 mm in diameter, hereinafter referred to as big head, which is articulated with a metal cup cemented or uncemented (2), whose inner surface is ultra-polished, similar to the cups utilized in metal-metal or double mobility implants, as shown in Figures 8 and 14. That is to say, the invention consists in a highly cross-linked polyethylene head (12A, 12B) that articulates with a metal cup (2).

In one embodiment of the invention, the highly cross-linked polyethylene head (12A) is part of a prosthesis to be used in total hip replacement, where the coupling mechanism to the femoral component is made by means of a metal core (17), stably assembled within the highly cross-linked polyethylene head (12A), using a firm locking mechanism (171). This metal core (17) contains inside the female counterpart of the Morse taper (14) to mate with the male counterpart (13) of the upper end of the femoral component, as shown in Figures 8 and 9.

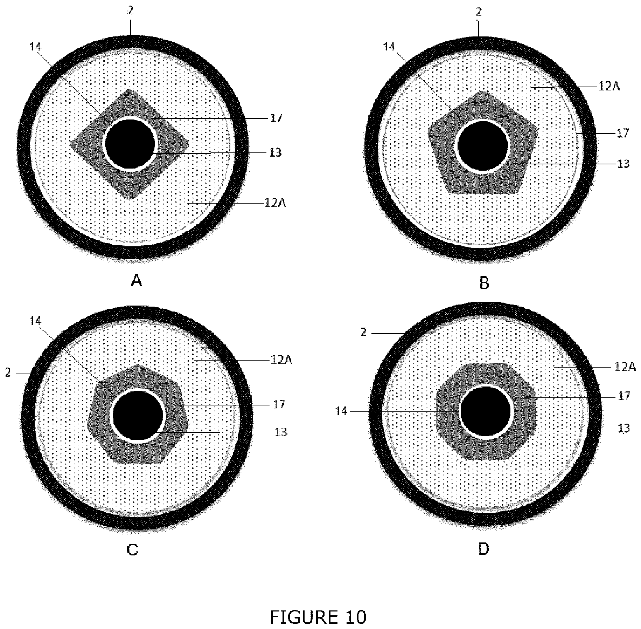

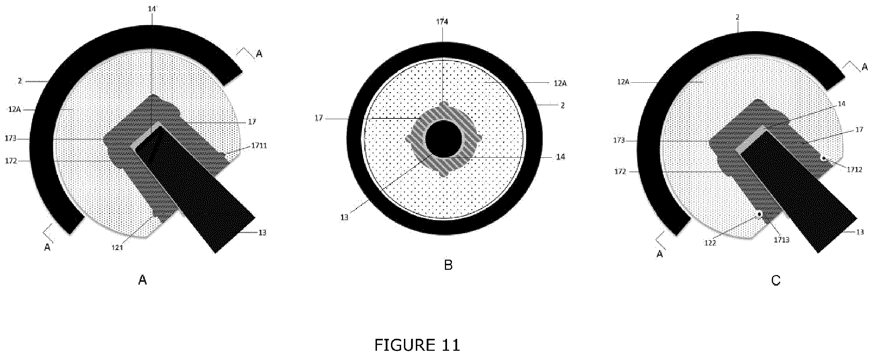

Such metal core (17) is characterized in that the shape of it is a truncated cone or pyramid, whose base is a spherical or a polyhedron selected from the group consisting of a square, pentagonal, hexagonal, heptagonal, octagonal shapes, etc., as illustrated in Figures 9, 10A, 10B, 10C and 10D. Even, the external shape of the metal core (17) may correspond to a truncated cone with a spherical base, as shown in Figures 11A, 11B and 11C. Regardless of the shape of said pyramid, the longitudinal section of the metal core (17) corresponds to a trapezoidal shape.

In one embodiment preferred of the invention, the metal core (17) has the form of a hexagonal truncated pyramid where the longitudinal section of the metal core (17) corresponds to a trapezoidal shape and the core has a hexagonal shape in the transverse plane, as evidenced in Figure 9. Said core (17) is coupled by press-fit into the polyethylene femoral head (12A), within an inner cavity of the same geometry. In a preferred embodiment, the metal core (17) is fixed inside the head (12A) using a locking mechanism (171) consisting of a flange which projects around of the external surface of the metal core (17) which is fitted in a slot (121) located in the internal space of the head (12A), as illustrated in Figure 8. In this manner rotational and longitudinal stability of the metal core (17) is achieved, avoiding movement and the production of wear particles. The shape and size of the core allows complete contact of the walls and roof with the inner polyethylene avoiding the presence of spaces that would allow deformation of the polyethylene under the loads.

In another alternative of the invention, the metal core (17) has a truncated cone shape, and the longitudinal section of the metal core (17) corresponds to a trapezoidal shape, but the core is shaped round in the transverse plane, as illustrated in the Figure 11A. In this case, the implant includes an anti-rotating mechanism (172), consisting of 1 to 6 longitudinal fins on the outer surface of the metal core (17), and of one longitudinal locking mechanism, which is selected from one perimeter beveled flange (1711) that fits into an also perimeter groove (121) in the interior space of the head (12A), shown in Figure 11A; or a metal ring (1712) which is located between a perimeter groove (1713) in the core (17) and a perimeter groove (122) in the interior space of the head (12A) represented in Figure 11 C.

In addition to the above features, the metal core (17) of the prosthesis, in any of its forms, always has rounded corners (173) to avoid areas of stress concentration as shown in Figures 8 to 12.

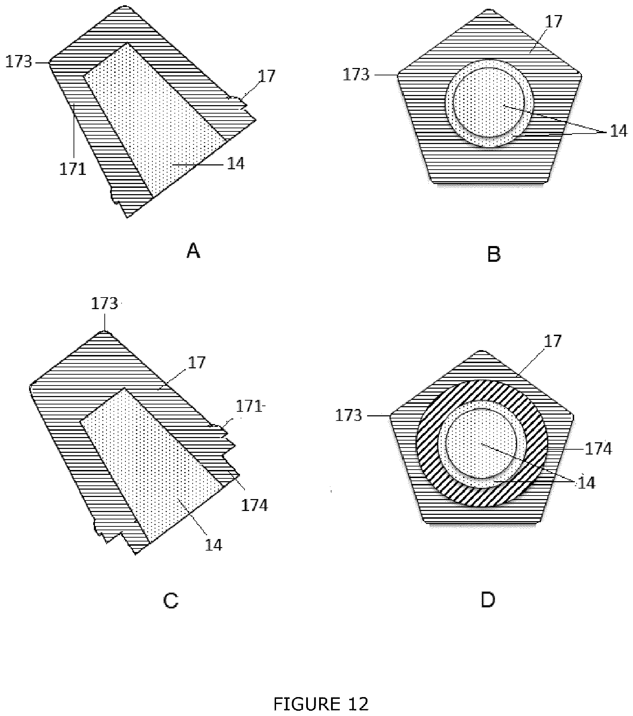

Likewise, the implant of the present invention may have an extension (174) of the female component (14) of the Morse taper, which extends beyond the inferior edge of the metallic core (17), as it is illustrated in Figures 12C and 12D. Such extension (174) is intended to improve to the versatility of the implant by allowing the length of the prosthetic femoral neck to be increased a bit more, similar to what in conventional metal heads is called skirted heads.

In order to provide proper stiffness to the implant, the walls of the metal core (17) should have a thickness between 4 mm and 8 mm to avoid deformations in its coupling to the femoral cone. Such core is made of a material selected from the group consisting of cobalt chromium alloys, titanium alloys or stainless steel, but can also be made of ceramic.

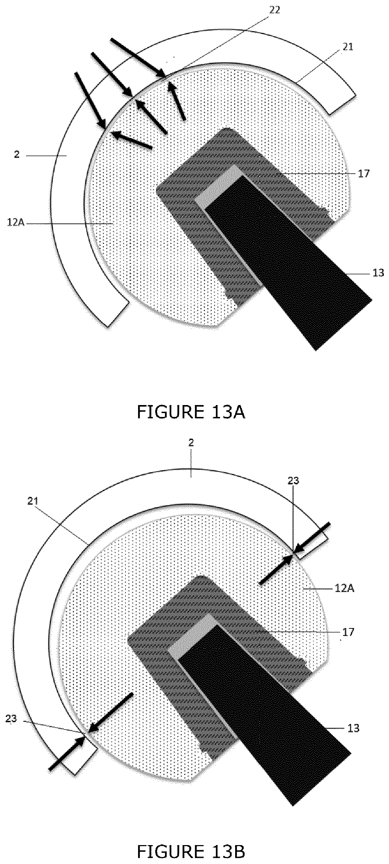

Regarding the radius of curvature of the polyethylene head (12A, 12B), it must have a clearance in relation to the internal diameter of the cup, between 50 and 150 microns, which allows a more polar (22) than equatorial (23) contact in the acetabular cup, as shown in Figure 13A. This, in order to avoid areas of increased friction in the equatorial area and obtain a better load transfer and lubrication.

Given the properties of the invention, the total hip replacement implant of the present invention has utility in the following situations:
1. Primary total hip replacement procedures, especially in older patients and/or those at high risk of dislocation.
2. The HCLPE head can be articulated, either with a solid metal uncemented cup; with an uncemented modular cup with a metal insert; or with cemented metal cups similar to the cups used for dual mobility.
3. Likewise, the prosthesis is ideal for total hip replacement revision surgeries, using either a metal cup without holes; modular cups with metal inserts; or with cemented metal cups with or without a reinforcing ring.
4. Likewise, the prosthesis can be used in large head metal on metal total hip replacement revision surgeries or in double mobility cup revision surgeries, preserving the original acetabular prosthetic component.
5. It can also be used in resurfacing replacement revision surgeries retaining the original acetabular prosthetic component.

In the other embodiment of the present invention, the prosthesis intended for hip resurfacing replacement is characterized in that the highly cross-linked polyethylene prosthetic femoral head (12B), with a diameter between 40 mm and 64 mm, has a lower extension or stem (15), which allows fixation directly to the femoral neck (16) with bone cement. Said femoral head (12B) articulates with a metallic acetabular component (2), ultra-polished inside, as shown in Figure 14A. In this embodiment of the invention, the fixation of the head to the femur is done with cement and the fixation of the acetabular component (2) may be cemented or uncemented, according to the design used.

In a preferred alternative of this embodiment, the extension or lower stem (15) of the prosthetic femoral head (12B) can be completely made of polyethylene or may be of polyethylene with an internal metal reinforcement (151), as illustrated in Figures 14A and 14B, respectively.

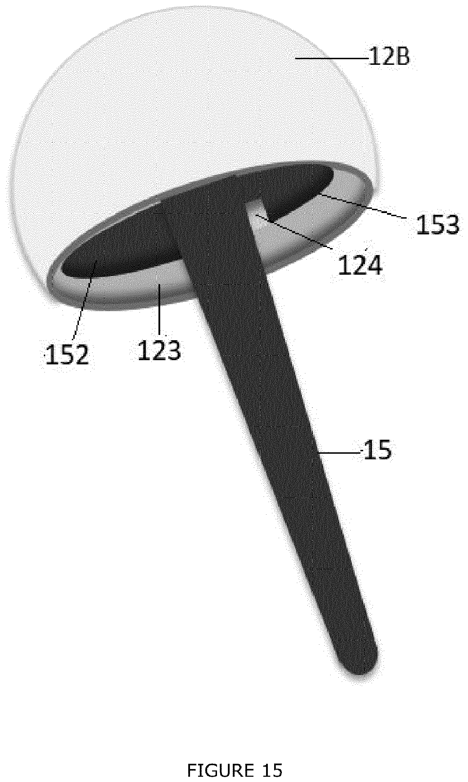

In a third alternative to the prosthesis intended for hip surface replacement, the implant of the invention comprises the polyethylene head (12B) with a thin metal back (152) attached to a metal stem (15), as shown in Figures 15, 16 and 17A. In order not to thin down the polyethylene, the metal backing (152) should have a thickness between 2 and 3 mm, preferably 2 mm.

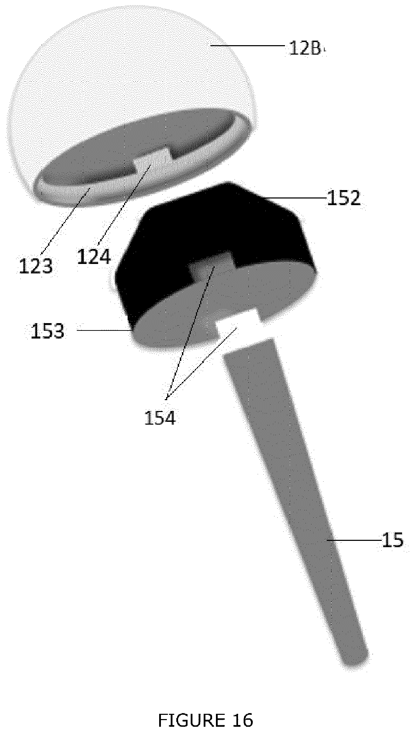

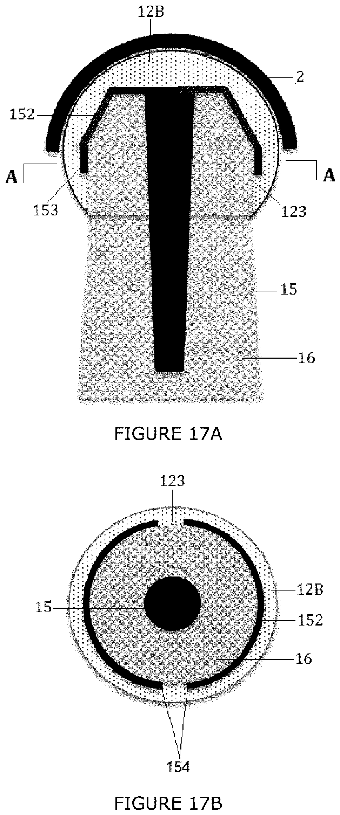

In this embodiment of the invention, the polyethylene head (12B) is secured to the metal support (152) by one rotational circumferential flange (123) located in its inside, which fits securely into the bottom edge (153) of the metal back (152), preventing the vertical movement of the head (12B). Likewise, the polyethylene head (12B) comprises 2 to 4 anti-rotating (124) tabs, preferably two tabs, which projects from the inner surface of the head (12B), as an extension of the upper end of the circumferential flange (123), one opposite the other, and fit in corresponding notches (154) of the lower edge (153) of the metal backing (152), to give stability to the polyethylene, as shown in Figures 16 and 17B.

The metallic stem and metal back can be made with an external porous cover for cementless use, or without a porous cover for use with cemented fixation.

For better rotational stability, the stem (15) used in the cemented fixation of the femoral component, in any of its modalities, is a taper, rectangular-shaped stem with rounded corners (not sharp), to avoid areas of stress in the cement mantle.

Likewise, the internal surface of the polyethylene head (12B) or the metal backing that will be in contact with the bone cement must have irregularities (grooves or squares not shown in the figures) for better inter-digitation of the cement and rotational stability.

Given the properties of the invention, the hip resurfacing replacement implant has utility in the following situations:
1. Primary hip replacement in young patients, without collapse of the femoral head, fo whom lengthening of the limb is not required.
2. Primary hip replacement in cases of proximal femur deformity due to osteotomy or old fracture.
3. Primary hip replacement in patients at high risk of dislocation.

In order to clearly define the scope of the present invention, some terms used in the description of the invention and facilitate its understanding are specified below.

*Press-fit.* It refers to the initial stability or grip mechanism of the implant, which provides the necessary stability to allow osteo-integration of the implant. It is generally performed by preparing a bed slightly smaller than the definitive implant, so that the latter enters in a tight way, preventing axial, angular and rotational movements.

*Hip joint.* It is the site of union between the pelvis and the femur. The spherical head of the femur fits into the acetabular socket of the pelvis and is the site of movement between the lower extremity and the pelvis. The term joint is also used to refer to the site of movement between the prosthetic head and acetabulum of the total hip replacement.

*Total Hip Replacement Surgery Revision.* A surgical procedure done to partially or completely change the components of a total hip replacement.

*Femoral component.* Refers to the prosthetic implant that is placed in the medullary canal of the superior end of the native femur. Generally, the prosthetic head is modular and adapts to the prosthetic stem using a Morse taper mechanism.

*Cemented femoral component (cemented femoral stem).* Refers to femoral prostheses made to be implanted using bone cement (poly methyl methacrylate). These implants have a smooth or slightly rough surface and their fixation is always done with bone cement.

*Uncemented femoral component (uncemented femoral stem).* This refers to the porous-coated femoral prosthesis for osteo-integration, which is implanted in the femur in total hip replacement.

*Morse taper.* It is the mechanism used to attach the prosthetic modular femoral head to the upper end or neck of the femoral stem. This ends in a segment in the form of a truncated cone (male), whose length is variable. The cone fits into the femoral head, which has a socket (female) with the same geometric shape and angulation. Its adjustment is made by impacting the head on the male component of the neck.

*Acetabular cup.* It is the component of a hip replacement prosthesis that is placed in the acetabular cavity of the pelvis. There are uncemented cups which are composed of a metallic cup with an external porous coating, generally hemispherical, and a modular component of polyethylene, ceramic or metal, which is assembled in the concavity of the cup. There are also cemented acetabular cups which are made entirely of polyethylene and are fixed to the bone with bone cement.

*Linear wear.* It refers to the magnitude of penetration of the prosthetic femoral head into the acetabular polyethylene or any other articular surface due to material wear. It is generally expressed in millimeters.

*Volumetric wear.* It is the term used to quantify the volume of material removed from the polyethylene or any other articular surface generated by movement and loads on the joint. The volume of polyethylene removed for an equal amount of penetration of the femoral head is greater in heads with a larger diameter.

*Debris.* These are the wear particles that are released on the movement surfaces of the prostheses. There are polyethylene, metal or ceramic debris.

*Intra-prosthetic dislocation.* It is a term used to describe the unique situation of dual mobility hip replacements in which the femoral head is decoupled from the polyethylene head. This occurs because the polyethylene containment mechanism is lost due to wear.

*Prosthetic dislocation.* It is the situation in which the femoral head of the total hip replacement is decoupled from the acetabular component when taken to extreme movements, since there is normally no mechanism for containing the head within the acetabular cavity.

*Modular.* Refers to prosthetic components made up of separate parts that are assembled during surgical implantation. For example, the acetabular component has an internal insert of polyethylene or ceramic that is assembled in the metal cup after placing it in the native acetabulum. This has two purposes, one is to allow the placement of the screws to fix the cup to the bone through holes in it and the other is to be able to use polyethylene with projections, eccentricity, lateralized as needed; or inserts made of ceramic or metal. Likewise, the prosthetic femoral head is modular with the body of the prosthesis, and its coupling is carried out by means of a Morse taper mechanism (male component - female component). This allows the length of the femoral neck to be modified according to the depth of the taper in the prosthetic head.

*Osteo-integration.* It refers to the process of bone growth on the porous covering of uncemented implants. When the bone growth on the cover is sufficient to provide stability to the implant, it is considered to be osteo-integ rated.

*Osteolysis.* It refers to the abnormal bone resorption process that occurs around prosthetic components, as a biological response to the deposit of polyethylene or metallic particles resulting from wear and to local changes in the pressure of peri-prosthetic fluids. As the bone is resorbed, the implant is left without structural support and the possibility of failure increases.

*Highly Cross-linked Polyethylene (HCLPE).* refers to a polymeric material made of high molecular weight polyethylene, which has been subjected to a process of irradiation with gamma rays or electron beams and heating, in order to produce crosslinking of the polymer chains. Both in hip simulators and in vivo wear measurements, a significant reduction in the amount of wear has been shown when used in the acetabular components of the total hip replacement.

*Local corrosive processes.* It refers to the processes of galvanic and crevice corrosion that occur in the metal-metal modularity sites, which end up releasing corrosion ions and metal particles to peri-prosthetic fluids and tissues, generating a local biological reaction and also systemic repercussions (generalized or in distant organs).

*Porous coating.* It refers to the coating that uncemented implants have on the external surface that is in contact with the native bone. There are several types of porous coatings and their function is to allow bone growth within the pores or on the irregularities of its surface, to give implants long-term stability.

*Peri-prosthetic tissues.* It is used to refer to the bone and soft tissues adjacent to the implant, both in the femur and in the pelvis. The wear debris of the materials of the joint replacements are mainly deposited in these tissues.

*Clearance.* It refers to the difference in the radius of curvature of the acetabular cavity and the femoral head of the prosthesis. Low tolerance joints increase friction on the surface and make lubrication difficult.

## Claims

1. Prosthesis for hip replacement **characterized in that** it comprises a highly cross-linked polyethylene head (12A and 12B) of 38 mm to 64 mm in diameter, and a cemented or uncemented metal cup (2), whose inner surface is ultra-polished.

2. The prosthesis according to claim 1, **characterized in that** it is a prosthesis for total replacement or is a prosthesis intended for hip resurfacing replacement.

3. The prosthesis according to claim 2, **characterized in that** it is a total replacement prosthesis, where the assembly of the highly cross-linked polyethylene head (12A) to the stem (11) of the femoral component is carried out by means of a metal core (17), which is fixed by a locking mechanism (171) inside of the polyethylene head (12A) and which contains the female counterpart (14) of the Morse taper to be coupled with male counterpart (13) located at the end upper part of the femoral component.

4. The prosthesis according to claim 3, **characterized in that** the metal core (17) has the shape of truncated cone or pyramid, whose base is a circle or a polyhedron and the longitudinal section of the metal core (17) corresponds to a trapezoidal shape.

5. The prosthesis according to claim 4, **characterized in that** the polyhedron is selected from the group consisting of a square, pentagonal, hexagonal, heptagonal or octagonal shape.

6. The prosthesis according to claim 5, **characterized in that** the metal core (17) has the shape of a truncated hexagonal pyramid.

7. The prosthesis according to claim 4, **characterized in that** the metal core (17) is fitted inside an internal cavity in the polyethylene femoral head (12A), which has the same geometry of the metal core (17), and is fixed in said head (12A) by a perimeter chamfered flange (1711), which projects on the outer surface of the metal core (17) and is inserted into a slot, also perimeter (121), located in the internal space of the head (12A).

8. The prosthesis according to claim 4, **characterized in that** the metal core (17) is fitted inside an internal cavity in the polyethylene femoral head (12A), which has the same shape of the metal core (17), and is fixed in said head (12A) by means of a perimeter wire (1712) that fits into a also perimeter grooves (122, 1713), located in the internal space of the head (12A) and on the external surface of the metal core (17), respectively.

9. The prosthesis according to one of the claims 4 to 8, **characterized in that** the metal core (17) has a truncated cone shape and includes an anti-rotating (172) mechanism, consisting of 1 to 6 longitudinal fins on the outer surface of the metallic core (17).

10. The prosthesis according to any one of claims 4 to 9, **characterized in that** the walls of the metal core (17) must have a thickness between 4 mm and 8 mm.

11. The prosthesis according to any one of claims 4 to 10, **characterized in that** the corners (173) of the metal core (17) are rounded.

12. The prosthesis according to any one of claims 4 to 11, **characterized in that** the metal core (17) is made of a material selected from the group consisting of ceramic or cobalt-chromium alloys, titanium alloys or stainless steel.

13. The prosthesis according to any one of claims 4 to 12, **characterized in that** the female component (14) of the Morse taper has an extension (174) that goes beyond the lower edge of the metal core (17).

14. The prosthesis according to any one of claims 1 to 13 , **characterized in that** the radius of curvature of the polyethylene head (12A) is between 50 and 150 microns less than the radius of curvature (21) of the interior of the metal cup (2) and the contact of the head (12A, 12B) in the acetabulum (2) is more polar (22) than equatorial (23).

15. The prosthesis according to claim 2, **characterized in that** it is a prosthesis intended hip resurfacing replacement and comprises a prosthetic femoral head (12B) made of highly cross-linked polyethylene, with a diameter between 40 mm and 64 mm, with a lower extension or stem (15) that is fixed to the femoral neck (16), and a metallic acetabular component (2) ultra-polished inside.

16. The prosthesis according to claim 15, **characterized in that** the fixation of the head (12B) to the femoral neck (16) takes place with bone cement and the fixation of the acetabular component (2) may be cemented or uncemented.

17. The prosthesis according to claim 15, **characterized in that** the lower extension or stem (15) of the prosthetic femoral head (12B) is made of polyethylene or polyethylene with an internal metal reinforcement (151).

18. The prosthesis according to claim 15, **characterized in that** the head (12B) is mounted on a metal-back (152) including a metal stem (15) and said metal-back (152) is secured to the head (12B) by a circumferential flange (123), located inside the polyethylene head (12B), which fits securely on the lower edge (153) of the metal backing (152).

19. The prosthesis according to claim 18, **characterized in that** the metal backing (152) has a thickness of 2 mm to 3 mm.

20. The prosthesis according to claim 19, **characterized in that** the metal back (152) has a thickness of 2 mm.

21. The prosthesis according to claim 18, **characterized in that** it further comprises between 2 to 4 anti-rotational tabs (124), which project from the inner surface of the head (12B), as an extension of the upper end of the circumferential flange (123), each one opposite to the other, and fit into the corresponding notches (154) of the lower edge (153) of the metallic support (152).

22. The prosthesis according to claim 21, **characterized in that** it comprises 2 opposite anti-rotational tabs (124) and 2 notches (154).
